# EUROPEAN PATENT SPECIFICATION

(11) **EP 4 438 111 B1**
(45) Date of publication and mention of the grant of the patent: **02.12.2026**
(21) Application number: 24166231.1
(22) Date of filing: 26.03.2024
(51) Int. Cl.: A61N 5/10

(54) **SYSTEMS AND METHODS FOR AUTOMATIC NORMAL TISSUE OBJECTIVES IN STEREOTACTIC BODY RADIOTHERAPY**
SYSTEME UND VERFAHREN FÜR AUTOMATISCHE NORMALGEWEBEZIELE IN DER STEREOTAKTISCHEN KÖRPERSTRAHLENTHERAPIE
SYSTÈMES ET PROCÉDÉS D'OBJECTIFS AUTOMATIQUES DE TISSU NORMAL EN RADIOTHÉRAPIE CORPORELLE STÉRÉOTAXIQUE

(30) Priority: 31.03.2023 US 202318129364
(43) Date of publication of application: 02.10.2024
(73) Proprietor: Siemens Healthineers International AG, 6312 Steinhausen (CH)
(72) Inventor: KUUSELA, Esa, 02320 Espoo (FI); MAKKONEN, Jarmo, 00520 Helsinki (FI); KORHONEN, Laura, 02340 Espoo (FI); VALENZUELA, Daniel, 0440 Helsinki (FI)
(74) Representative: Mathisen & Macara LLP

(56) References cited:
- EP-A1- 3 530 319
- EP-B1- 3 573 715
- US-A1- 2018 078 792

## Description

### FIELD OF THE DISCLOSURE

The present application relates generally to systems and methods for automatically defining and handling normal tissue objectives in radiotherapy planning. Specifically, the present application relates to automatically generating normal tissue objectives for optimizing a radiotherapy plan in stereotactic body radiotherapy.

### BACKGROUND

Radiotherapy is a radiation-based therapy that is used as a cancer treatment. Specifically, high doses of radiation are used to kill or shrink a tumor. The target region of a patient's body that is intended to receive radiation (e.g., tumor) is referred to as the planning target volume (PTV). The goal is to deliver enough radiation to the PTV to kill the cancerous cells. However, other organs that are adjacent to, or surrounding, the PTV can be in the way of radiation beams and can receive enough radiation to damage or harm such organs or anatomical regions. These organs or anatomical regions are referred to as organs at risk (OARs). Usually a physician, a radiologist or an oncologist identifies both the PTV and the OARs prior to radiotherapy using, for example, computed tomography (CT) images, magnetic resonance imaging (MRI) images, positron emission tomography (PET) images, images obtained via some other imaging modality, or a combination thereof. For instance, the physician or the radiologist may manually mark the PTV and/or the OARs on the medical images of the patient.

Using the medical images of the patient as well as the identified PTV and the OARs, a treatment planner determines the radiation parameters to be used during the radiotherapy treatment. These radiation parameters include, for example, the type, the angle, the radiation intensity and/or the shape of each radiation beam. In determining these parameters, the treatment planner attempts to achieve a radiation dose distribution to be delivered to the patient that meets predefined criteria, e.g., set by the oncologist or the radiologist. Such criteria usually include predefined radiation dose thresholds or ranges for the PTV and the OARs to be met.

To optimize the radiation parameters in a way to meet the predefined criteria, the treatment planner usually runs a plurality of simulations with various radiation parameters, and selects a final set of radiation parameters to be used based on the simulation results. This process usually involves tweaking parameters for the treatment plan optimization after each simulation. Such approach is time consuming, tedious and may not provide optimal results. For instance, a patient can wait for days or weeks before a radiation therapy plan specific to the patient is ready.

Document EP 3 530 319 A1 describes methods and systems for planning and optimizing treatment sessions of a patient in radiotherapy systems. Document US 2018 078 792 A1 discloses a method for treatment planning for radiation therapy.

### SUMMARY

Embodiments described herein relate to improving radiotherapy treatment planning by controlling the radiation dose for normal tissue outside PTV(s). Normal tissue objectives (NTOs) can be defined to enforce rapid decrease of the radiation dose outside the PTV(s) and to prevent the formation of hotspots. However, achieving this goal can be technically challenging, especially for Stereotactic Body Radiotherapy (SBRT) where radiation fields are typically coplanar. Embodiments described herein provide solutions for this technical problem.

According to one aspect, a method of radiation treatment planning is disclosed in independent claim 1. Further embodiments of the method of radiation treatment planning are disclosed in its dependent claims.

According to another aspect, a radiation treatment planning system is disclosed in independent claim 7. Further embodiments of the radiation treatment planning system are disclosed in its dependent claims.

According to yet another aspect, a non-transitory computer-readable medium is disclosed in independent claim 12.

### BRIEF DESCRIPTION OF THE DRAWINGS

**FIG. 1** shows a block diagram illustrating an example computer environment for implementing methods and processes described herein, according to an embodiment.
**FIGS. 2A-2C** show various plots illustrating different normal tissue objectives, according to example embodiments.
**FIG. 3** shows a diagram depicting virtual structures defined in association with a target or PTV, according to inventive concepts of this disclosure.
**FIG. 4** shows a diagram illustrating of defining and using dose distribution to implement distribution-based NTO, according to inventive concepts of this disclosure.
**FIG. 5** is a flowchart illustrating a method of radiation treatment planning, according to an example embodiment.

Some or all of the figures are schematic representations for purposes of illustration. The foregoing information and the following detailed description include illustrative examples of various aspects and implementations, and provide an overview or framework for understanding the nature and character of the claimed aspects and implementations. The drawings provide illustration and a further understanding of the various aspects and implementations, and are incorporated in and constitute a part of this specification.

### DETAILED DESCRIPTION

Following below are more detailed descriptions of various concepts related to, and implementations of, methods, apparatuses, and systems for radiation treatment planning. The various concepts introduced above and discussed in greater detail below may be implemented in any of numerous ways as the described concepts are not limited to any particular manner of implementation. Examples of specific implementations and applications are provided primarily for illustrative purposes.

Radiotherapy treatment planning (also referred to herein as radiation treatment planning) is a complex and patient specific optimization problem. Given the anatomy of the patient, e.g., as illustrated in medical images of the patient, identifications or masks of the PTV and the OARs and clinical objectives set by physicians, the goal is to determine a treatment plan that satisfies the clinical objectives. The radiation treatment planning problem is usually formulated as an optimization problem with a cost function (also referred to herein as an objective function) defined in terms of one or more optimization objectives (also referred to herein as objective terms). The optimization objectives can include one or more objective terms related to constraints to be satisfied within the PTV, one or more objective terms related to constraints to be satisfied within the OARs, and one or more objective terms related to constraints to be satisfied within normal tissue.

When a photon optimizer is used for creating a radiation therapy treatment plan, the cost function to be optimized can be constructed by considering the clinical goals for defined PTVs or target structures (e.g., regions where some therapeutic dose is requested) and for OARs. In addition, the cost function may further include objective terms reflecting or indicative of constraints (e.g., radiation dose constraints) related to the normal tissue. As used herein, normal tissue refers to anatomical regions or parts of the body that are not part of the PTVs or the OARs. The objective terms reflecting or indicative of constraints related to the normal tissue are referred to herein as normal tissue objectives (NTOs). The purpose of using NTOs in the cost function to be optimized is to control the final optimized radiation treatment plan in a way to avoid exceptionally high radiation doses in places where they are not expected (e.g., in normal tissue regions).

In Stereotactic Body Radiotherapy (SBRT), it may be desired and important to enforce a fast radiation dose fall-off outside of the PTVs without compromising the PTV dose conformity and dose coverage. SBRT employs highly focused radiation beams to apply high radiation doses to the area to be treated. A slow decrease in the radiation dose when moving away from PTVs may cause significant damage to normal tissue, which in turn can lead to significant and permanent side effects. By enforcing fast radiation dose fall-off outside of the PTVs and avoiding unexpectedly large dose peaks elsewhere in the normal tissue, the damage to the healthy tissues around the PTVs can be reduced or minimized. Controlling the radiation dose outside the PTVs calls for new normal tissue objectives (NTOs) that take into consideration field geometry that is specific to SBRT. In SBRT, a co-planar orientation of radiation beams is employed where the centerlines of all the radiation beams lie in a same plane referred to as the 'co-plane.' The co-planar radiation beams lead to a co-planar field geometry that creates radiation dose distributions with significantly slower fall-off in the co-plane than in directions perpendicular or transverse to the co-plane. In other words, the radiation field is consistently much stronger along the co-plane and falls off fast along the direction perpendicular to the co-plane.

In the current disclosure, a novel NTO approach that performs well in SBRT cases as well as other scenarios, and enforces fast radiation dose fall-off in the outside vicinity of PTVs without compromising the PTV dose conformity and dose coverage is described. By integrating the NTOs described herein into an objective function and optimizing (e.g., minimizing the objective function, radiotherapy treatment plans having fast radiation dose fall-off outside PTVs, PTV dose conformity and no significant radiation dose excess in unexpected locations can be achieved. The NTO approach described herein can be viewed as a combination of (i) a voxel-dose (or curve-based) approach, where a target dose fall-off curve is employed and voxels with radiation doses exceeding the set curve are penalized relative to the excess dose at that location, and (ii) a distribution-based approach where dose distributions within given distance-to-target intervals are used and any voxel-dose that is an outlier to the corresponding distribution (with pre-defined degree) is penalized. Also, the targeted dose level in the voxel-dose based part of the NTOs described herein can be set to be challenging rather than to enforce it to be strictly met or satisfied everywhere.

Systems and methods described herein produce radiotherapy treatment plans which enforce rapid decrease in radiation dose outside the PTVs, especially in the case of SBRT. While the systems and methods as well as the NTOs describe herein are discussed in relation with SBRT, these solutions are not limited to SBRT and can be used in other types of radiation therapy.

For purposes of reading the description of the various embodiments below, the following disclosure describes a computing and network environment for radiation treatment planning which may be useful for practicing embodiments described herein and describes systems and methods for automatic normal tissue objectives.

### Computing and Network Environment for Radiation Treatment Planning

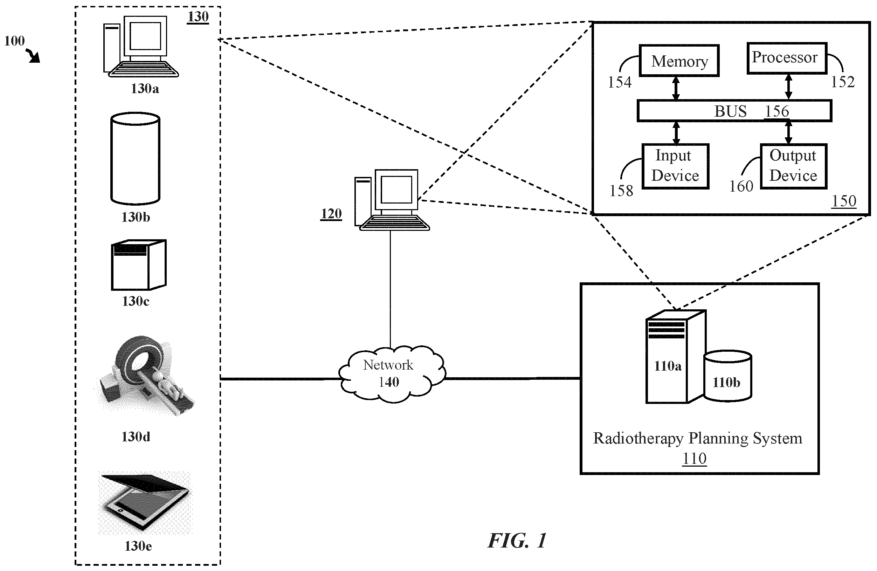

**FIG.** 1 illustrates an example computer environment 100 that can be used to provide a network-based implementation of the methods described herein. The computer environment **100** can include a radiotherapy planning system **110** (also referred to herein as radiation treatment system **110**), a user computing device **120** and electronic data sources **130a-e** (referred to collectively as electronic data source **130**). The radiotherapy planning system **110** can include one or more computer servers **110a** (or more generally one or more computing devices **110a**) and one or more databases **110b.** The above-mentioned components may be connected to each other through a network **140.** The examples of the network **140** may include, but are not limited to, private or public LAN, WLAN, MAN, WAN, and the Internet. The network **140** may include both wired and wireless communications according to one or more standards and/or via one or more transport mediums.

The communication over the network **140** may be performed in accordance with various communication protocols such as Transmission Control Protocol and Internet Protocol (TCP/IP), User Datagram Protocol (UDP), and IEEE communication protocols. In one example, the network **140** may include wireless communications according to Bluetooth specification sets or another standard or proprietary wireless communication protocol. In another example, the network **140** may also include communications over a cellular network, including, e.g., a GSM (Global System for Mobile Communications), CDMA (Code Division Multiple Access), EDGE (Enhanced Data for Global Evolution) network.

The computer environment **100** is not necessarily confined to the components described herein and may include additional or alternate components, not shown for brevity, which are to be considered within the scope of the embodiments described herein.

In some implementations, the computer server **110a** can be configured to execute computer instructions to perform any of the methods described herein or operations thereof. The computer server(s) **110a** may generate and display an electronic platform to display information indicative of, or related to, parameters of a radiation treatment plan and/or parameter values related to the objective function used to determine the radiation treatment plan or objective terms thereof. The electronic platform may include graphical user interface (GUI) displayed on the user computing device **120.** An example of the electronic platform generated and hosted by the computer server(s) **110a** may be a web-based application or a website configured to be displayed on different electronic devices, such as mobile devices, tablets, personal computer, and the like (e.g., user computing device **120**).

The computer server(s) **110a** may host a website accessible to end-users, where the content presented via the various webpages may be controlled based upon each particular user's role or viewing permissions. The computer server(s) **110a** may be any computing device comprising a processor and non-transitory machine-readable storage capable of executing the various tasks and processes described herein. Non-limiting examples of such computing devices may include workstation computers, laptop computers, server computers, laptop computers, and the like. While the computer environment **100** in **FIG. 1** includes a single computer server **110a**, in some configurations, the radiotherapy planning system **110** may include any number of computing devices, e.g., operating in a distributed computing environment.

The computer server(s) **110a** may execute software applications configured to display the electronic platform (e.g., host a website), which may generate and serve various webpages to each user computing device **120.** Different users operating the user computing device(s) **120** may use the website to view and/or interact with the output radiation treatment plans, or input parameter values and/or constraints used to generate or define the objective function, e.g., clinical goals, weighting coefficients for different objective terms of the objective function, parameters of shell regions or combination thereof, among others.

In some implementations, the computer server **110a** may be configured to require user authentication based upon a set of user authorization credentials (e.g., username, password, biometrics, cryptographic certificate, and the like). In such implementations, the computer server(s) **110a** may access the system database(s) **110b** configured to store user credentials, which the computer server(s) **110a** may be configured to reference in order to determine whether a set of entered credentials (purportedly authenticating the user) match an appropriate set of credentials that identify and authenticate the user.

In some configurations, the computer server(s) **110a** may generate and host webpages based upon a particular user's role (e.g., administrator, employee, and/or bidder). In such implementations, the user's role may be defined by data fields and input fields in user records stored in the system database(s) **110b.** The computer server(s) **110a** may authenticate the user and may identify the user's role by executing an access directory protocol (e.g. LDAP). The computer server(s) **110a** may generate webpage content that is customized according to the user's role defined by the user record in the system database(s) **110b.**

In some embodiments, the computer server(s) **110a** receives medical images, masks and/or medical data indicative of medical goals from a user (or retrieve from a data repository), process the data, and displays an indication of the treatment trajectory on the electronic platform. For instance, in a non-limiting example, a user operating the computing device **130a** uploads a series of images of a CT scan or other medical images using the electronic platform. The computer server(s) **110a** can determine the treatment trajectory based on input data, and display the results via the electronic platform on the user computing device **120** or the computing device **130a.** The user computing device **120** and/or the computing device **130a** may be any computing device comprising a processor and a non-transitory machine-readable storage medium capable of performing the various tasks and processes described herein. Non-limiting examples of a network node may be a workstation computer, laptop computer, tablet computer, and server computer. In operation, various users may use user computing devices **120** and or computing device **130a** to access the GUI operationally managed by the computer server(s) **110a.**

The electronic data sources **130** may represent various electronic data sources that contain and/or retrieve medical images of patients. For instance, database 130b and third-party server **130c** may represent data sources providing the corpus of data (e.g., medical images, masks or other medical data) needed for the computer server(s) **110a** to determine radiation treatment plans. The computer server(s) **110a** may also retrieve the data directly from a medical scanner **130e** and/or medical imaging device **130d** (e.g., CT scan machine).

In some implementations, the methods described herein or operations thereof can be implemented by the user device **120,** any of the electronic devices **130** or a combination thereof.

While **FIG**. **1** shows a network based implementation, it is to be noted that methods described herein can be implemented by a single computing device that receives the medical images and medical data for a patient and determines a radiation treatment trajectory or path according to methods described herein.

The computing system **150** in **FIG. 1** depicts an example implementation of a system architecture for any of the computing devices **110a, 120** and/or **130a-e.** For instance, the computing system **150** can include, but is not limited to, a computed tomography (CT) scanner, a medical linear accelerator device, a desktop, a laptop, a hardware computer server, a workstation, a personal digital assistant, a mobile computing device, a smart phone, a tablet, or other type of computing device. The computing system **150** can include one or more processors **152** to execute computer code instructions, a memory **154** and a bus **156** communicatively coupling the processor **152** and the memory **154.**

The one or more processors **152** can include a microprocessor, a general purpose processor, a multi-core processor, a digital signal processor (DSP) or a field programmable gate array (FPGA), an application-specific integrated circuit (ASIC) or other type of processor. The one or more processors **152** can be communicatively coupled to the bus **156** for processing information. The memory **154** can include a main memory device, such as a random-access memory (RAM) or other dynamic storage device, coupled to the bus **156** for storing information and instructions to be executed by the processor(s) **152.** The main memory device can be used for storing temporary variables or other intermediate information during execution of instructions (e.g., related to methods described herein such as method **300**) by the processor(s) **152.** The computing system **150** can include a read-only memory (ROM) or other static storage device coupled to the bus **156** for storing static information and instructions for the processor(s) **152.** For instance, the ROM can store medical images or other medical data of patients, for example, received as input. The ROM can store computer code instructions related to, or representing an implementation of, methods described herein. A storage device, such as a solid state device, magnetic disk or optical disk, can be coupled to the bus **156** for storing (or providing as input) information and/or instructions.

The computing system **150** can be communicatively coupled to, or can include, an input device **158** and/or an output device **160.** The computing system **150** can be coupled via the bus **156** to the output device **160.** The output device **160** can include a display device, such as a Liquid Crystal Display (LCD), Thin-Film-Transistor LCD (TFT), an Organic Light Emitting Diode (OLED) display, LED display, Electronic Paper display, Plasma Display Panel (PDP), or other display, etc., for displaying information to a user. The output device **160** can include a communication interface for communicating information to other external devices. An input device **158,** such as a keyboard including alphanumeric and other keys, may be coupled to the bus **156** for communicating information and command selections to the processor **154.** In some implementations, the input device **158** may be integrated within a display device, such as in a touch screen display. The input device **158** can include a cursor control, such as a mouse, a trackball, or cursor direction keys, for communicating direction information and command selections to the processor 124 and for controlling cursor movement on the display device.

According to various implementations, the methods described herein or respective operations can be implemented as an arrangement of computer code instructions that are executed by the processor(s) **152** of the computing system **150.** The arrangement of computer code instructions can be read into main memory device from another computer-readable medium, such as the ROM or the storage device. Execution of the arrangement of computer code instructions stored in main memory device **154** can cause the computing system **150** to perform the methods described herein or operations thereof. In some implementations, one or more processors **152** in a multi-processor arrangement may be employed to execute the computer code instructions representing an implementation of methods or processes described herein. In some other implementations, hard-wired circuitry may be used in place of or in combination with software instructions to effect illustrative implementation of the methods described herein or operations thereof. In general, implementations are not limited to any specific combination of hardware circuitry and software. The functional operations described in this specification can be implemented in other types of digital electronic circuitry, in computer software, firmware, hardware or a combination thereof.

In some implementations, the computing system **150** can include a plurality of computing devices, e.g., operating in a distributed computing environment. The computing system **150** can represent an example implementation of the radiotherapy planning system **110.**

### Automatic Normal Tissue Objectives

Controlling the radiation dose for normal tissue in radiotherapy treatment planning can be achieved by incorporating additional objective terms within the objective function to be optimized (e.g., minimized or maximized depending on how the objective function is defined). As mentioned above, such additional objective terms can be referred to as normal tissue objective (NTO) terms or normal tissue objectives (NTOs), and can be used to enforce constraints specific to the normal tissue. The "NTO" can be used interchangeably to indicate the object or goal to be achieved or the corresponding objective term in the objective function. One goal or constraint that may be desired for normal tissue is to enforce relatively rapid decrease of the radiation dose in the outside vicinity of PTVs while ensuring PTV dose conformity and dose coverage. Another goal is to limit dose peaks or hot spots elsewhere in the normal tissue. However, achieving these goals calls for careful design of the corresponding NTOs. For instance, attempting to implement a strict radiation dose fall-off and/or to limit dose peaks in the normal tissue can lead to poor target coverage. Also, in the case of SBRT where the radiation fields are typically or commonly co-planar, the resulting radiotherapy treatment plans may not produce fast decreasing radiation dose along the co-plane, which in turns leads to significant damage to normal tissue of the patient.

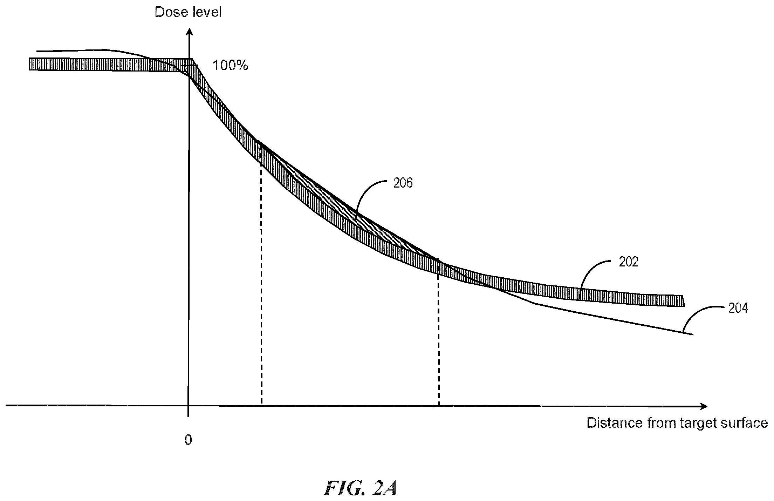

Referring now to **FIGS 2A-2C****,** various plots illustrating different normal tissue objectives are shown, according to example embodiments. In **FIG. 2A****,** a first NTO is depicted or expressed as a desired radiation dose function **202.** The first NTO is constructed or designed based on the idea that a "realistic" dose fall-off curve **202** as a function of distance from a PTV is created or defined. One or more objective terms to enforce the radiation dose outside the PTV to be equal to or below the curve 202 can be used in the objective function to be optimized. During the optimization of the objective function, the radiotherapy planning system **110** or the processor(s) **152** applies heavy penalty to any voxel (or voxel dose value) where the radiation dose value exceeds the expected radiation dose value from the set curve **202.** For instance, a voxel that is at a distance d from the PTV will be penalized if the voxel radiation dose exceeds the value of the curve **202** at the distance d. In **FIG. 2A****,** the curve **204** represents an example radiation dose curve of a radiotherapy treatment plan that could be determined during the optimization process. The region **206** between the curves **202** and **204** represents the set of voxels (set of voxels located within the distance interval between the dashed lines) and the corresponding radiation dose values at which penalty will be applied.

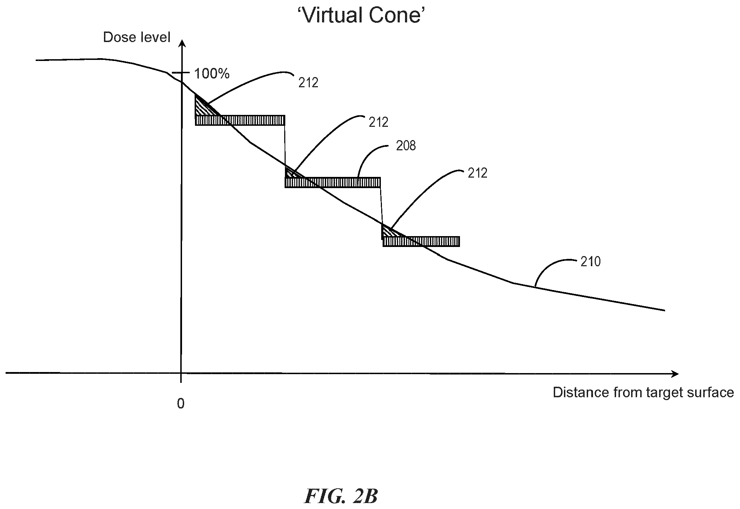

In **FIG. 2B****,** a second NTO is depicted or expressed in terms of a desired or reference radiation dose function **208.** The shape of the radiation dose fall-off curve **208** can be viewed as a virtual cone or a two-dimensional (2-D) step function. The 'virtual-cone' approach depicted in **FIG. 2B** includes multiple shell-structures (e.g., concentric shell structures) defined around the PTV where each shell-structure has its own target dose level. The 'virtual-cone' NTO is constructed or designed based on the idea that the 2-D step function represents a good approximation of the radiation dose fall-off outside the PTV. An objective term to enforce the radiation dose outside the PTV to be equal to or below the curve **208** can be used in the objective function to be optimized. During the optimization of the objective function, the radiotherapy planning system **110** or the processor(s) **152** applies heavy penalty to any voxel (or voxel dose value) where the current voxel radiation dose value exceeds the expected radiation dose value from the set curve **208.** For instance, the curve **210** represents an example radiation dose curve of a radiotherapy treatment plan that could be determined during the optimization process, and the regions **212** represent the set of voxels and the corresponding radiation dose values at which penalty will be applied.

Referring now to **FIG**. **2C**, a third NTO approach is illustrated or expressed in terms of a desired or reference radiation dose function **214,** according to at least one example embodiment. The radiation dose function **214** is defined in terms of two virtual shell structures (per each PTV). The radiotherapy planning system **110** or the processor(s) **152** can define or construct a separate radiation dose objective for each of the two virtual shell structures (e.g., a first radiation dose objective for shell 1 and a second radiation dose objective for shell 2). Instead of selecting goal dose levels or distributions based on what is assumed to be achievable, the radiotherapy planning system **110** or the processor(s) **152** can define or determine a desired or reference dose level for each virtual shell structure based on the dose level(s) monitored in the PTV according to input clinical goals. For instance, the radiotherapy planning system **110** or the processor(s) **152** can define or determine a first fraction or percentage of the PTV desired radiation dose level to be achieved in the first virtual shell structure, and define or determine a second fraction or percentage of the PTV desired radiation dose level to be achieved in the second virtual shell structure. In general, a separate target dose can be defined or assigned to each of the first and second virtual shell structures.

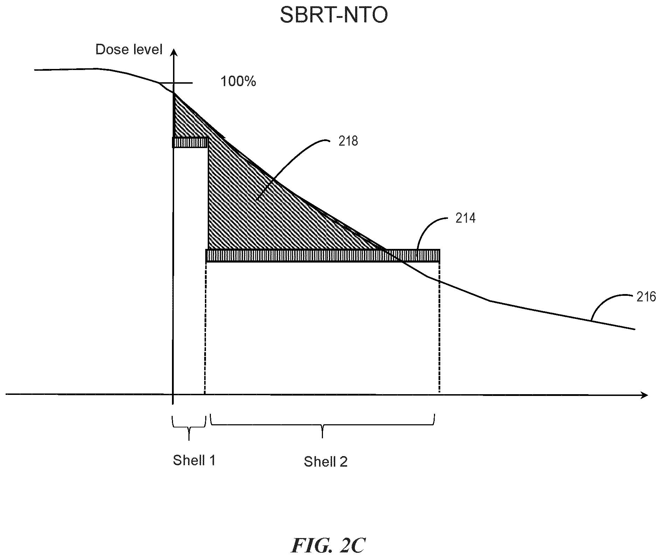

In **FIG. 2C****,** the curve **216** represents an example radiation dose curve of a radiotherapy treatment plan that could be determined during the optimization process, and the region 218 represent the set of voxels and the corresponding radiation dose values at which penalty will be applied. The region **218** of penalized voxel doses is larger than the regions **216** and **212** of FIGS. **2A** and **2B****.** The radiotherapy planning system **110** or the processor(s) **152** can compensate the relatively large region **218** of penalized voxel doses by applying relatively weaker penalty strength. For example, the radiotherapy planning system **110** or the processor(s) **152** can assign lower weights to objective terms corresponding to the NTO objectives for shell 1 and shell 2 compared to weight assigned to the PTV objective. Furthermore, the radiotherapy planning system **110** or the processor(s) **152** can use different weighting coefficients for shell 1 and shell 2. For example, the radiotherapy planning system **110** or the processor(s) **152** can use a smaller weighting coefficient for shell 1 which is closer to the PTV, than the weighting coefficient for shell 2.

The third NTO approach is referred to herein as SBRT-NTO, and it can include one or more other NTO terms to enforce voxel-dose within another region, other than the first and second virtual shell structures, to be in accordance with a predefined distribution. In other words, the additional NTO term(s) can penalize voxel-doses deviating away from the predefined distribution. The region where the radiation dose is enforced to follow the predefined distribution is different from the first virtual structure and the second virtual structure.

It is to be noted that the x-axis in **FIGS. 2A-2C** represents the distance from the PTV and the y-axis represents the dose level. The region to the left of the y-axis corresponds to the PTV. The SBRT-NTO approach was found to outperform other NTO approaches in the case of SBRT where the radiation field is typically co-planar.

In the third NTO approach, a separate pair of virtual shell structures (or virtual shell regions) can be defined around each PTV. More generally, one or more separate virtual shell structures can be defined around each PTV. A separate target radiation dose can be assigned or specified for each virtual shell structure. In some implementations, the NTO according to the third NTO approach can be defined as: $\begin{matrix}{C}_{NTO} = {\sum }_{j = 1}^{N} \left(\frac{{w}_{1 j}}{2}{\sum }_{i ∈ {S}_{1 j}} max {\left(0,D\left({x}_{i}\right)-{D}_{shell , 1 j}\right)}^{2} + \frac{{w}_{2 j}}{2} {\sum }_{i ∈ {S}_{2 j}} max {\left(0,D\left({x}_{i}\right)-{D}_{shell , 2 j}\right)}^{2}\right) \\ + \frac{{w}_{d}}{2} {\sum }_{i ∈ Ω / {∪}_{j = 1}^{N} {s}_{3 j}^{′}} max {\left(0,D\left({x}_{i}\right)-{D}_{dist}\left({x}_{i}\right)\right)}^{2} .\end{matrix}$ (1) In equation (1) above, Ω represents the total anatomical region considered in the planning process, N represents the total number of PTVs in Ω, and j represents the PTV index. For each PTV with index *j*, the first and second virtual shell structures are denoted as *S*_{1*j*} and *S*_{2*j*}, respectively. Also, for the PTV with index *j*, ${S}_{3 j}^{′}$ represents another structure or region associated with the predefined distribution

The anatomical region Ω can be divided into small sub-volumes (e.g., voxels or groups of voxels) of equal size, indexed by *i.* The center of each sub-volume with index *i* is denoted as *xᵢ* and the radiation dose of the sub-volume is denoted as *D*(*xᵢ*)*.* Each term max(0, *D*(*xᵢ*) *- D*_{*shell,*1*j*})² is used to force radiation doses of sub-volumes within virtual shell structure *S₁ⱼ* to be smaller than a radiation dose level *D*_{*shell,*1*j*} that is specific to the virtual shell structure *S₁ⱼ*. Also, each term max(0, *D*(*xᵢ*) *- D*_{*shell,*2*j*})² is used to force radiation doses of sub-volumes within each virtual shell structure *S₂ⱼ* to be smaller than a radiation dose level *D*_{*shell,*2*j*} that is specific to the virtual shell structure *S₂ⱼ*. Also, separate weight parameters (or weighting coefficients) *w₁ⱼ* and *w₂ⱼ* can be assigned to the sum of the terms max(0, *D*(*xᵢ*) *- D*_{*shell,*2*j*})² and the sum of the terms max(0, *D*(*xᵢ*) - *D*_{*shell,*1*j*})², respectively.

The last summation term in equation (1) represents a distribution-based NTO to cause the radiation dose within a given region to follow one or more distributions. The last summation is done over all sub-volumes *i* in the distribution-based NTO related region defined as the anatomical region Ω without any of the regions ${S}_{3 j}^{′}$. Each region ${S}_{3 j}^{′}$ can include the PTV with index *j* and the corresponding shell structures *S*_{1*j*} and *S*_{2*j*}, and the distribution related NTO term (e.g., last summation term of equation (1)) applies outside the regions ${S}_{3 j}^{′}$. Within each sub-volume with index *i* that belongs to $Ω / {∪}_{j = 1}^{N} {S}_{3 j}^{′}$, the target or desired dose level is *D_{dist}*(*xᵢ*) can be determined using, or based on, a given distribution. In some implementations, each region ${S}_{3 j}^{′}$ can be associated with a corresponding distribution, and the distribution used to determine the target or desired dose level *D_{dist}*(*xᵢ*) can be the distribution associated with the region ${S}_{3 j}^{′}$ that is closet to the sub-volume center *xᵢ.* For example, the distances between each sub-volume center *xᵢ* and the regions ${S}_{3 j}^{′}$ can be calculated, and the sub-volume *i* can be associated with the region ${S}_{3 j}^{′}$ closest to center *xᵢ* or with the corresponding distribution. In some implementations, the desired or target dose level *D_{dist}*(*xᵢ*) for the sub-volume *i* can be determined or defined as a predefined measure of the distribution (e.g., the dose value of certain percentile) associated with the closest ${S}_{3 j}^{′}$. A weight parameter (or weighting coefficient) *w_{d}* can be applied to the distribution-based NTO term (e.g., last summation term in equation (1).

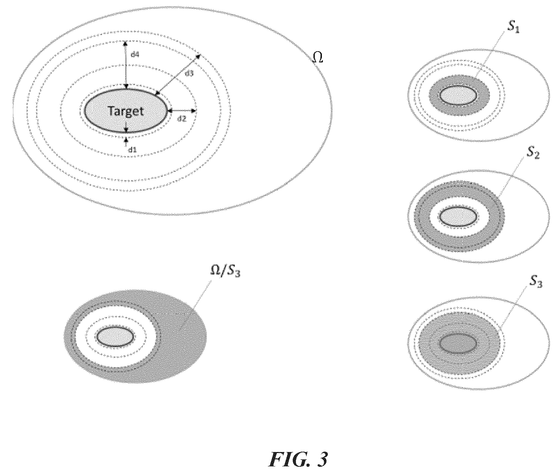

Referring now to **FIG. 3**, a diagram depicting virtual structures defined in association with a target or PTV are shown, according to inventive concepts of this disclosure. The shell structure *S*_{1*j*} can be defined immediately around the PTV. In some implementations, there may be a gap region (e.g., a relatively thin or small region) between the PTV and virtual shell structure *S*_{1*j*}. The virtual shell structure *S*_{2*j*} can be defined immediately around the virtual shell structure *S*_{1*j*}. The region ${S}_{3 j}^{′}$ can be defined as the union of the target or PTV, the shell structures, e.g., *S*_{1*j*} and *S*_{2*j*}, defined around the PTV, and gaps in between (if any) as depicted in **FIG. 3****.** In some implementations, the region ${S}_{3 j}^{′}$ can be a smaller (or larger) volume than the union of the PTV, *S*₁*ⱼ, S*_{2*j*} and any gaps in between. For example, if the region ${S}_{3 j}^{′}$ is smaller than the union, then the region $Ω / {∪}_{j = 1}^{N} {S}_{3 j}^{′}$ of the distribution-based cost term will overlap with at least virtual shell structure *S*_{2*j*}. If the region ${S}_{3 j}^{′}$ is larger than the union then there will be some gap between the region $Ω / {∪}_{j = 1}^{N} {S}_{3 j}^{′}$ of the distribution-based cost term and the virtual shell structure *S*_{2*j*}. In some implementations, the region ${S}_{3 j}^{′}$ can be defined as an extension of the target or PTV that includes voxels where the distance to the nearest target or PTV voxel is smaller than a preset value. The preset value can be smaller or larger than what was used to define the outer rim of the virtual shell structure *S*₂*ⱼ.*

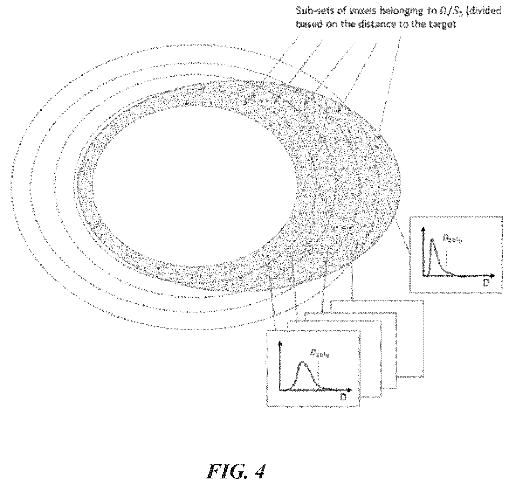

Referring now to **FIG. 4**, a diagram illustrating of defining and using dose distribution to implement distribution-based NTO, according to inventive concepts of this disclosure. In general, the dose distribution can be defined in various ways, and the approach illustrated in **FIG. 4** represents an example implementation. There is a lot of leeway in how the distribution can be defined for use to implement the distribution-based NTO. As depicted in **FIG. 4****,** the distribution-based NTO related region $Ω / {∪}_{j = 1}^{N} {S}_{3 j}^{′}$ can be divided into sub-sets based on the distance to nearest PTV voxel. Inside each sub-set some, the radiotherapy planning system 110 or the processor(s) thereof can calculate a corresponding statistical measure (or statistical metric), such as the mean dose, or certain percentile. The radiotherapy planning system 110 or the processor(s) thereof can penalize any voxel (belonging to given sub-set) if the radiation dose value of the voxel is larger than the statistical measure (or another threshold value derived from the statistical measure in a predefined way) of the sub-set. For example, *D_{dist}*(*xᵢ*) can be defined as ${D}_{dist} \left({x}_{i}\right) = {a}_{l} {D}_{mean}^{l}$, where *xᵢ* belongs to sub-set with index *l, aₗ* is a predefined coefficient, and ${D}_{mean}^{l}$ is the mean dose of voxels belonging to sub-set with index *l.* It is to be noted that that the division of the region $Ω / {∪}_{j = 1}^{N} {S}_{3 j}^{′}$ to sub-sets can be coarse or fine to lead either a relatively small or large number of sub-sets. Also, while **FIG. 4** shows a single PTV, the same concept can be applied for multiple PTVs or targets.

In some implementations, when optimizing *C_{NTO}* (as part of optimizing the full complete objective function or cost function) iteratively, at each iteration, the radiotherapy planning system 110 or the processor(s) thereof can calculate parameters of the distribution (or statistical measures) using the current dose matrix (e.g., in certain sub-volume of the patient) determined or calculated at the same iteration. The radiotherapy planning system 110 or the processor(s) thereof can penalize individual voxel-doses if they exceed certain threshold determined using one or more distribution parameters or statistical metrics. In other words, the distribution parameters or statistical metrics used can be predefined, but the radiation dose values (or dose matrix or matrices) used to calculate the distribution parameters can vary from one iteration to another. The distribution parameters or statistical metrics can be re-calculated in every iteration based on the latest radiation dose matrix.

In equation (1), *C_{NTO}* represents an example total NTO to implement or enforce constraints related to normal tissue. The full complete objective function (or cost function) can be described as: $\begin{matrix}C = {C}_{NTO} + {\sum }_{j = 1}^{N} \left(\frac{{w}_{TL , j}}{2}{\sum }_{i ∈ {PTV}_{j}} min {\left(0,D\left({x}_{i}\right)-{D}_{Presc , j}\right)}^{2}\right) \\ + {\sum }_{j = 1}^{N} \left(\frac{{w}_{TU , j}}{2}{\sum }_{i ∈ {PTV}_{j}} max {\left(0,D\left({x}_{i}\right)-{D}_{max , j}\right)}^{2}\right) \\ + {\sum }_{k = 1}^{M} \left(\frac{{w}_{OAR , k}}{2}{\sum }_{i ∈ {OAR}_{k}} max {\left(0,D\left({x}_{i}\right)-{D}_{dist}^{{OAR}_{k}}\left({x}_{i}\right)\right)}^{2}\right) .\end{matrix}$ (2) The first summation term in equation (2) represents the total lower-end objective for the PTVs in Ω, while the second summation term in equation (2) represents the total upper-end objective for the PTVs in Ω. The total lower-end objective relates to constraints enforcing minimum dose levels within the PTVs, and the total upper-end objective relates to constraints enforcing maximum dose levels within the PTVs. For instance, *D_{presc,j}* represents the target dose level for *PTVⱼ,* which can be viewed as the minimum radiation dose level to be achieved for *PTVⱼ.* The dose level *D_{max,j}* represents the highest or maximum accepted dose level for *PTVⱼ.* The last summation term represents penalties that can be applied to radiation dose values within OARs. In some implementations, the objective summation term related to the OARs can include distribution-based terms. For example, the dose level ${D}_{dist}^{{OAR}_{k}} \left({x}_{i}\right)$ can be determined or defined as a predefined measure (e.g., a mean or a given percentile) of a distribution associated with *OARₖ* where k represents an index of OARs.

Different weight parameters (or weighting coefficients) *w_{TL,j}, w_{TU,j}* and *w_{OAR,k}* can be assigned or applied to the penalty terms in equation (2). The weight parameter *w_{TL,j}* can be applied to the lower-end penalty term for *PTVⱼ,* the weight parameter *w_{TU,j}* can be applied to the upper-end penalty term for *PTVⱼ,* and the weight parameter *w_{OAR,k}* can be applied to the penalty term for *OARₖ.* In some implementations, the user can assign different priorities (e.g., different numeric priority value) for each of the PTVs, each of the OARs and/or the normal tissue. The radiotherapy planning system **110** or the processor(s) **152** can determine the weight parameters *w_{TL,j}, w_{TU,j}, w_{OAR,k}, w₁ⱼ, w₂ⱼ* and/or *w_{d}* using (or based on) the input priority values. The priorities can determine roughly how much the various penalty terms in equation (1) and/or equation (2) should contribute to the total cost *C*. In some implementations, the radiotherapy planning system **110** or the processor(s) **152** can down scale the weight parameters *w₁ⱼ, w₂ⱼ* and/or *w_{d}* associated with the normal tissue, e.g., relative to weighting coefficients associated with the PTVs and/or OARs, to prevent the *C_{NTO}* from dominating the total cost *C* given that the value of *C_{NTO}* may be relatively large. In some implementations, the radiotherapy planning system **110** or the processor(s) **152** can determine the weight parameters *w_{TL,j}, w_{TU,j}, w_{OAR,k}, w₁ⱼ, w₂ⱼ* and/or *w_{d}* differently (e.g., other than using input priority values). In some implementations, the radiotherapy planning system **110** or the processor(s) **152** can select or determine the weight parameters *w₁ⱼ, w₂ⱼ* and/or *w_{d}* associated with the normal tissue to be relatively smaller compared to weighting coefficients associated with the PTVs and/or OARs.

It is to be noted that the objective formulations in equations (1) and (2) depict an example implementation of the NTO and the total objective function. Other formulations can be used. For instance, the penalty terms associated with the virtual shell structures *S*_{1*j*} and *S*_{2*j*} and/or the distribution-based penalty terms in equation (2) can be defined differently. Also, the penalty terms associated with the PTVs and/or OARs can be defined differently.

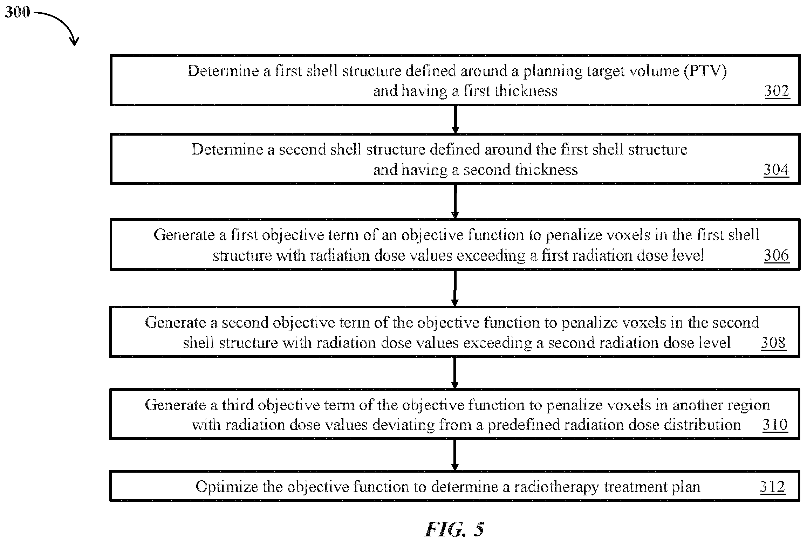

Referring now to **FIG. 5****,** a flowchart illustrating a method **300** of radiation treatment planning is shown, according to inventive concepts of this disclosure. In brief overview, the method **300** can include the radiotherapy planning system **110** or the processor(s) **152** determining a first virtual shell structure around a PTV and having a first thickness (STEP **302**), and determining a second virtual shell structure around the first virtual shell structure and having a second thickness (STEP **304**). The method **300** can include radiotherapy planning system **110** or the processor(s) **152** generating a first objective term, of an objective function, penalizing voxels in the first virtual shell structure with radiation dose values exceeding (or deviating from) a first radiation dose level (STEP **306**), and generating a second objective term, of the objective function, penalizing voxels in the second virtual shell structure with radiation dose values exceeding (or deviating from) a second radiation dose level (STEP **308**). The method 300 can include radiotherapy planning system **110** or the processor(s) **152** generating a third objective term, of the objective function, penalizing voxels in another region, different from the first and second virtual shell structures, with radiation dose values deviating from a predefined distribution (STEP **310**), and optimizing the objective function to determine a radiotherapy treatment plan (STEP **312**).

The method 300 can include the radiotherapy planning system **110** or the processor(s) **152** determining a first virtual shell structure (or shell region) around a PTV and having a first thickness (STEP **302**), and determining a second virtual shell structure (or shell region) around the first virtual shell structure and having a second thickness (STEP **304**). The radiotherapy planning system **110** or the processor(s) **152** can receive medical images indicative of an anatomical region of a patient including one or more target regions (e.g., one or more PTVs) to be radiated. The medical images can include a mask image indicative of the PTV(s). In some implementations, the radiotherapy planning system **110** or the processor(s) **152** can determine the PTV(s), for example, using indications (e.g., manual marking on the image(s) to indicate the PTV(s)) in one or more medical images. The radiotherapy planning system **110** or the processor(s) **152** can employ segmentation algorithms to determine or refine the PTV image region(s).

The radiotherapy planning system **110** or the processor(s) **152** can receive information indicative of a spatial scale of the received medical image(s). The spatial scale can indicate the actual dimensions (in the patient's anatomical region) of the image pixels or voxels. Using the spatial scale, the radiotherapy planning system **110** or the processor(s) **152** can determine the first thickness of the first virtual shell structure and the second thickness of the second virtual shell structure in voxels. The radiotherapy planning system **110** or the processor(s) **152** can define or construct the first virtual shell structure to have the first thickness, for example, by defining a first number of single-voxel layers around the PTV. The radiotherapy planning system **110** or the processor(s) **152** can define or construct the second virtual structure to have the second thickness, for example, by defining a second number of single-voxel layers around the first virtual shell structure.

In some implementations, the second thickness can be greater than the first thickness. In some implementations, the sum of the first thickness and the second thickness can be equal to about 2 centimeters (e.g., ± 5%). In some implementations, the first thickness can be equal to about 3 millimeters (e.g., ± 5%). In some implementations, the second thickness can be equal to about 1.7 centimeters (e.g., ± 5%). In some implementations, the radiotherapy planning system **110** or the processor(s) **152** can define or determine the first thickness, the second thickness or the sum thereof based on the size of the PTV. For example, the radiotherapy planning system **110** or the processor(s) **152** can use a lookup table or some predefined formulation to determine the first thickness, the second thickness or the sum thereof based on the size of the PTV. It is to be noted that the radiotherapy planning system **110** or the processor(s) **152** can define or construct, for each PTV, a corresponding pair of shell structures with a first virtual shell structure defined around the PTV and having a first thickness or depth and a second shell structured defined around the first shell structure and having a second thickness or depth.

In some implementations, the radiotherapy planning system **110** or the processor(s) **152** can define or construct a single virtual shell structure or more than two virtual shell structures around the PTV. In general, the radiotherapy planning system **110** or the processor(s) **152** can define or construct one or more corresponding shell structures (or one or more shell regions) around each PTV within the anatomical region Ω considered (e.g., imaged) for planning the radiation therapy plan.

The method **300** can include radiotherapy planning system **110** or the processor(s) **152** generating a first objective term, of an objective function, penalizing radiation therapy values in the first virtual shell structure exceeding a first radiation dose level (STEP 306), and generating a second objective term, of the objective function, penalizing radiation therapy values in the second virtual shell structure exceeding a second radiation dose level (STEP 308). The first radiation dose level can be specific to the first shell structure. The second radiation dose level can be specific to the second shell structure. In some implementations, the radiotherapy planning system **110** or the processor(s) **152** can determine the first radiation dose level and/or the second radiation dose level using one or more predefined radiation dose levels of the PTV.

For example, the radiotherapy planning system **110** or the processor(s) **152** can receive one or more clinical objectives or goals for each PTV (or for all PTVs). The radiotherapy planning system **110** or the processor(s) **152** can also receive one or more clinical objectives or goals for each OAR. For example, the clinical objectives for the PTV(s) can include the objectives D90.0% ≥ 50.00 Gy, D99.0% ≥ 46.50 Gy and D20.0% < 55.00 Gy. The constraint that D90.0% ≥ 50.00 Gy means that the radiation dose received by 90% of the PTV is greater than or equal to 50.00 Gy. The constraint that D99.0% ≥ 46.50 Gy means that the radiation dose received by 99% of the PTV is greater than or equal to 46.50 Gy. The constraint that D20.0% < 55.00 Gy means that less than 20% of the PTV receives a radiation dose smaller than 55.00 Gy. The radiotherapy planning system **110** or the processor(s) **152** can determine or define the first and/or second radiation dose levels using, for example, the lowest target radiation dose or the prescription dose for the PTV(s). Considering the example clinical objectives for the PTV(s), the prescription radiation dose *D_{Presc}* for the PTV(s) can be equal to 46.50 Gy.

The radiotherapy planning system **110** or the processor(s) **152** can define radiation dose constraints for each of the virtual shell structures using the prescription dose *D_{Presc}* for the PTV. For example, the radiotherapy planning system **110** or the processor(s) **152** can define or construct a first constraint for the first virtual shell structure as: D_{shell,1} ≤ α₁ D_{Presc}, where D_{shell,1} represents the radiation dose within the first shell structure and α1 represents a first fraction or first percentage. For example, α1 can be equal to about 80% (e.g., ± 5%). The radiotherapy planning system **110** or the processor(s) **152** can define or construct a second constraint for the second virtual shell structure as: D_{shell,2} ≤ α₂ D_{Presc}, where D_{shell,2} represents the radiation dose within the second shell structure and α₂ represents a second fraction or second percentage. For example, α₂ can be equal to about 40% (e.g., ± 5%). The radiotherapy planning system **110** or the processor(s) **152** can define or determine α₁ and α₂ such that α₁ ≥ α₂. The radiotherapy planning system **110** or the processor(s) **152** can define or determine the first radiation dose level as α₁ D_{Presc}, can define or determine the second radiation dose level as α₂ D_{presc}. In some implementations, the maximum dose level of all lower objectives associated with the PTV can be used to determine the first and/or second radiation dose level(s).

The radiotherapy planning system **110** or the processor(s) **152** can determine or construct, for each of the virtual shell structures, a corresponding NTO term to be incorporated in the objective function to be optimized. The radiotherapy planning system 110 or the processor(s) **152** can determine or construct a first NTO term to enforce the first NTO within the first shell structure, and a second NTO term to enforce the second NTO within the second shell structure. For example, radiotherapy planning system 110 or the processor(s) **152** can determine or construct the first NTO term as ∑_{i∈*S*1} max(0, *D*(*xᵢ*) *- D*_{*Shell*,1})² and determine or construct the second NTO term as Σ_{i∈*S*2} max(0, *D* (*xᵢ*) *- D*_{*shell,*2})² where *S*₁ and *S*₂ represent the first and second shell structures, respectively, as described in relation with equation (1). In the case where the anatomical region Ω includes multiple PTVs, the radiotherapy planning system **110** or the processor(s) **152** can define a pair of shell structures around each PTV, and determine or construct a corresponding NTO for each shell structure, as described in above in relation with equation (1). In general, the radiotherapy planning system **110** or the processor(s) **152** can define one or more shell structures around each PTV, and determine or construct a corresponding NTO for each shell structure to penalize radiation dose values in the shell structure that exceed a radiation dose level specific to, or associated with, the shell structure. In other words, any number of shell structures can be defined around a given PTV. The number of shell structures defined can vary from one PTV to another or can be the same for all PTVs.

The method 300 can include radiotherapy planning system **110** or the processor(s) **152** generating a third objective term, of the objective function, penalizing radiation dose values in another region, different from the first and second virtual shell structures, deviating from a predefined radiation dose distribution (STEP **310**). The radiation dose distribution can be determined based on simulation data and/or imaging data of a plurality of patients. The radiation dose distribution can describe statistically how the radiation dose is varied within the PTV and/or outside the PTV. In some implementations, a separate distribution can be defined or determined for each PTV or for each shell region including a corresponding PTV, such as the shell regions ${S}_{3 j}^{′}$ described above in relation with equation (1). The third objective term can be viewed as a distribution related NTO term that is used to force the radiation dose to conform with some radiation dose distribution or with a distribution parameter.

As discussed in relation with equation (1), a separate radiation dose distribution can be defined in relation with each PTV or with each predefined region, e.g., ${S}_{3 j}^{′}$, including a corresponding PTV. The radiotherapy planning system **110** or the processor(s) **152** can apply the third objective term outside the predefined regions, e.g., ${S}_{3 j}^{′}$, including corresponding PTVs. At each point or sub-volume outside the predefined regions ${S}_{3 j}^{′}$, the radiotherapy planning system **110** or the processor(s) **152** can determine the closest (in distance) region ${S}_{3 j}^{′}$ and use the dose distribution corresponding to the closest region ${S}_{3 j}^{′}$*.* For instance, referring back to equation (1), *D_{dist}*(*xᵢ*) can be defined or determine as a parameter or metric, such as a mean or a given percentile, of the dose distribution associated with the region ${S}_{3 j}^{′}$ that is closest to the center *xᵢ* of the sub-volume *i*. As used herein, a percentile metric or measure of a dose distribution represents a radiation dose value achieved or exceeded by a given percent of the volume (or voxels) of a given region. For example, *D_{dist}*(*xᵢ*) can be equal to a dose level associated with some dose distribution at which certain percentage of a region volume would have radiation dose values exceeding or equal to the dose level representing a certain percentile parameter or metric of the dose distribution. In general, the distribution related objective term penalizes radiation dose values within a normal tissue region (e.g., defined as outside the regions ${S}_{3 j}^{′}$) that are deviating from one or more radiation dose distributions.

The radiotherapy planning system **110** or the processor(s) **152** can determine or construct an objective function including the first NTO term, the second NTO term and the third NTO term. The objective function can include one or more objective terms for one or more PTVs. The objective function can include one or more objective terms for one or more OARs. As discussed above, equation (2) provides an example of an objective function that includes NTO terms as described herein. The radiotherapy planning system **110** or the processor(s) **152** can assign weighting coefficients, such as *w_{TL,j}, w_{TU,j}, w_{OAR,k}, w₁ⱼ, w₂ⱼ* and/or *w_{d}*, to the various objective terms of the objective function. The radiotherapy planning system **110** or the processor(s) **152** can assign smaller weighting coefficients to objective terms (or optimization terms) corresponding to the NTOs for shell 1 and shell 2 than the weighting coefficients associated with objective terms corresponding to PTV constraints or goals. For each PTV, the radiotherapy planning system **110** or the processor(s) **152** can assign a smaller coefficient for the corresponding shell 1, which is closer to the PTV, than the weighting coefficient for shell 2. The weighting coefficient can be determined or defined as discussed above in relation with equations (1) and (2).

In some implementations, the radiotherapy planning system **110** or the processor(s) **152** can determine or construct a general spatial objective to control the radiation dose in normal tissue relatively far from the PTV(s) (e.g., outside the defined virtual shell structures). The general spatial objective can be a distribution based objective. The use of such additional normal tissue objective allows for handling (or avoiding) unexpectedly high dose regions further away from PTV(s). The radiotherapy planning system **110** or the processor(s) **152** can determine or define the additional normal tissue objective to control the radiation dose at a distance of 10 mm or more from any PTV.

In some implementations, a pair or more of PTVs may be close to each other resulting in the corresponding virtual shells overlapping with one another. For example, a second shell for a first PTV may overlap with another second shell of a second PTV, with a first virtual of the second PTV or even with the second PTV. The radiotherapy planning system **110** or the processor(s) **152** can determine an overlapping region (or each overlapping region) and penalize voxels (or voxel dose values) in the overlapping region with radiation dose levels exceeding the maximum target radiation dose in the overlapping region. For example, in an overlapping region between a second shell for a first PTV and another second shell of a second PTV, the radiotherapy planning system **110** or the processor(s) **152** can employ the objective term corresponding to the highest target radiation dose among the objective term of the second shell for the first PTV and the objective term of the second shell for the second PTV. In an overlapping region between a second shell for a first PTV and a first shell of a second PTV, the radiotherapy planning system **110** or the processor(s) **152** can employ the objective term corresponding to the first shell of the second PTV because it has the highest target radiation dose. Also, in an overlapping region between a second shell for a first PTV and a second PTV, the radiotherapy planning system **110** or the processor(s) 152 can employ the objective term corresponding to the second PTV because it has the highest target radiation dose.

In some implementations, the radiotherapy planning system **110** or the processor(s) **152** can determine or define an additional virtual shell structure between a PTV and the corresponding first shell structure. The radiotherapy planning system **110** or the processor(s) **152** can enforce no objective or goal within this additional shell structure.

The method **300** can include radiotherapy planning system **110** or the processor(s) **152** optimizing the objective function to determine a radiotherapy treatment plan (STEP **310**). The radiotherapy planning system **110** or the processor(s) **152** can minimize (or maximize) the objective function, e.g., iteratively, to determine a radiotherapy treatment plan that satisfies (at least to some extent) the input clinical conditions and the automatically defined or constructed NTOs. It is to be noted that the various embodiments, implementations or features described in relation with **FIGS. 1-4** are also applicable to method **300.** In other words, method **300** can be implemented to incorporate (or according to any of) embodiments, implementations or features described in relation with **FIGS. 1-4****.**

It is to be noted that the SBRT NTO approach described herein can be employed in both coplanar and non-coplanar radiation field arrangements. For example, the SBRT NTO approach can be used for both IMRT and VMAT plans, and can be applied to all treatment sites. While other NTO approaches may perform better in strongly non-coplanar field geometry, the SBRT NTO was found to outperform other NTO approaches especially in coplanar radiation field arrangements.

Each method described in this disclosure can be carried out by computer code instructions stored on computer-readable medium. The computer code instructions, when executed by one or more processors of a computing device, can cause the computing device to perform that method.

While this disclosure contains many specific embodiment details, these should not be construed as limitations on the scope of any inventions or of what may be claimed, but rather as descriptions of features specific to particular embodiments of particular inventions. Certain features described in this specification in the context of separate embodiments can also be implemented in combination in a single embodiment. Conversely, various features described in the context of a single embodiment can also be implemented in multiple embodiments separately or in any suitable subcombination. Moreover, although features may be described above as acting in certain combinations and even initially claimed as such, one or more features from a claimed combination can in some cases be excised from the combination, and the claimed combination may be directed to a subcombination or variation of a subcombination.

Similarly, while operations are depicted in the drawings in a particular order, this should not be understood as requiring that such operations be performed in the particular order shown or in sequential order, or that all illustrated operations be performed, to achieve desirable results. In certain circumstances, multitasking and parallel processing may be advantageous. Moreover, the separation of various system components in the embodiments described above should not be understood as requiring such separation in all embodiments, and it should be understood that the described program components and systems can generally be integrated in a single software product or packaged into multiple software products.

References to "or" may be construed as inclusive so that any terms described using "or" may indicate any of a single, more than one, and all of the described terms.

Thus, particular embodiments of the subject matter have been described. Other embodiments are within the scope of the following claims. In some cases, the actions recited in the claims can be performed in a different order and still achieve desirable results. In addition, the processes depicted in the accompanying figures do not necessarily require the particular order shown, or sequential order, to achieve desirable results. In certain embodiments, multitasking and parallel processing may be advantageous.

## Claims

1. A method of radiation treatment planning comprising:
determining, by one or more processors (**152**), a first shell structure (S₁) defined around but not including a planning target volume, PTV, and having a first thickness;
determining, by the one or more processors (**152**)**,** a second shell structure (S₂) defined around but not including the first shell structure (S₁) and having a second thickness;
generating, by the one or more processors (**152**), a first objective term of an objective function for optimizing a radiotherapy treatment plan, the first objective term penalizing radiation dose values in the first shell structure (S₁) exceeding a first dose level specific to the first shell structure (S₁), the first objective term incorporating a first normal tissue objective, NTO, term, the first NTO term enforcing a first NTO within the first shell structure (S₁);
generating, by the one or more processors (**152**), a second objective term of the objective function, the second objective term penalizing radiation dose values in the second shell structure (S₂) exceeding a second dose level specific to the second shell structure (S₂), the second dose level smaller than the first dose level, the second objective term incorporating a second NTO term, the second NTO term enforcing the second NTO within the second shell structure (S₂);
generating, by the one or more processors (**152**), a third objective term of the objective function, the third objective term penalizing radiation dose values in a region, different from the first and second shell structures (S₁, S₂), deviating from a predefined dose distribution; and
optimizing, by the one or more processors (**152**), the objective function to determine the radiotherapy treatment plan,
wherein the region different from the first and second shell structures (S₁, S₂) includes a normal tissue region outside a third shell structure (S₃) defined around the PTV, and
wherein the third objective term penalizes the radiation dose values in the region different from the first and second shell structures (S₁, S₂) exceeding a parameter of the predefined dose distribution and wherein optionally:
the parameter of the predefined distribution includes:
a mean of the predefined distribution; or
a specific parameter of the predefined distribution.

2. The method of claim 1, wherein the second thickness is greater than the first thickness; or
the first thickness is equal to about 3 millimeters; or
the second thickness is equal to about 1.7 centimeters.

3. The method of any one of claim 1 or claim 2, wherein the PTV is a first PTV and the method further comprising:
determining, by the one or more processors (**152**), a third shell structure defined around but not including a second PTV and having a third thickness;
determining, by the one or more processors (**152**), a fourth shell structure defined around but not including the third shell structure and having a fourth thickness;
generating, by the one or more processors (**152**), a fourth objective term of the objective function, the fourth objective term penalizing radiation dose values in the third shell structure exceeding a third radiation dose level specific to the third shell structure; and
generating, by the one or more processors (**152**), a fifth objective term of the objective function, the fifth objective term penalizing radiation dose values in the fourth shell structure exceeding a fourth radiation dose level specific to the fourth shell structure, the fourth radiation dose level smaller than the third radiation dose level.

4. The method of claim 3, wherein the region includes a further region outside a fifth shell structure defined around the first PTV and outside a sixth shell structure defined around the second PTV,
the predefined dose distribution is a first dose distribution associated with the fifth shell structure, and a second dose distribution is associated with the sixth shell structure, and
the third objective term penalizes radiation dose values in the further region deviating from the first dose distribution or the second dose distribution.

5. The method of claim 3 or claim 4, wherein at least the second shell structure (S₂) and the fourth shell structure are overlapping and the method further comprising:
determining, by the one or more processors (**152**), an overlapping region of the second and fourth shell structures;
penalizing, by the one or more processors (**152**), radiation dose values in the overlapping region exceeding a maximum of the second radiation dose level and the fourth radiation dose level.

6. The method of any one of claims 1 to 5, wherein the objective function is optimized iteratively, and wherein generating the third objective term of the objective function includes, at each iteration:
calculating radiation dose values within the region different from the first and second shell structures (S₁, S₂); and
calculating a metric of the predefined dose distribution using the radiation dose values within the region different from the first and second shell structures (S₁, S₂), the metric of the predefined dose distribution used to determine which radiation dose values to be penalized.

7. A radiation treatment planning system (**150**) comprising:
one or more processors (**152**); and
a memory (**154**) to store computer code instructions, the computer code instructions when executed cause the one or more processors (**152**) to:
determine a first shell structure (S₁) defined around but not including a planning target volume, PTV, and having a first thickness;
determine a second shell structure (S₂) defined around but not including the first shell structure (S₁) and having a second thickness;
generate a first objective term of an objective function for optimizing a radiotherapy treatment plan, the first objective term penalizing radiation dose values in the first shell structure (S₁) exceeding a first radiation dose level specific to the first shell structure (S₁), the first objective term incorporating a first normal tissue objective, NTO, term, the first NTO term enforcing a first NTO within the first shell structure (S₁);
generate a second objective term of the objective function, the second objective term penalizing radiation dose values in the second shell structure (S₂) exceeding a second radiation dose level specific to the second shell structure (S₂), the second radiation dose level smaller than the first radiation dose level, the second objective term incorporating a second NTO term, the second NTO term enforcing the second NTO within the second shell structure (S₂);
generate a third objective term of the objective function, the third objective term penalizing radiation dose values in a region, different from the first and second shell structures (S₁, S₂), deviating from a predefined dose distribution; and
optimize the objective function to determine the radiotherapy treatment plan,
wherein the third objective term penalizes the radiation dose values in the region different from the first and second shell structures (S₁, S₂) exceeding a parameter of the predefined dose distribution and optionally
wherein the parameter of the predefined distribution includes:
a mean of the predefined distribution; or
a specific parameter of the predefined distribution.

8. The radiation treatment planning system (**150**) of claim 7,
wherein the second thickness is greater than the first thickness and optionally
wherein at least one of the following applies:
the first thickness is equal to about 3 millimeters; or
the second thickness is equal to about 1.7 centimeters.

9. The radiation treatment planning system (**150**) of claim 7 or claim 8, wherein the PTV is a first PTV and wherein the one or more processors (**152**) are further configured to:
determine a third shell structure defined around but not including a second PTV and having a third thickness;
determine a fourth shell structure defined around but not including the third shell structure and having a fourth thickness;
generate a fourth objective term of the objective function to penalize radiation dose values in the third shell structure exceeding a third radiation dose level specific to the third shell structure; and
generate a fifth objective term of the objective function, the fifth objective term penalizing radiation dose values in the fourth shell structure exceeding a fourth radiation dose level specific to the fourth shell structure, the fourth radiation dose level smaller than the third radiation dose level.

10. The radiation treatment planning system (**150**) of claim 9, wherein the region includes a region outside a fifth shell structure defined around the first PTV and outside a sixth shell structure defined around the second PTV,
the predefined dose distribution is a first dose distribution associated with the fifth shell structure, and a second dose distribution is associated with the sixth shell structure, and optionally wherein the PTV is a first PTV and wherein the third objective term penalizes radiation dose values in the region deviating from the first dose distribution or the second dose distribution.

11. The radiation treatment planning system (**150**) of any one of claims 7 to 10, wherein the objective function is optimized iteratively, and wherein in generating the third objective term of the objective function the one or more processors (**152**) are configured to, at each iteration:
calculate radiation dose values within the region different from the first and second shell structures (S₁, S₂); and
calculate a metric of the predefined dose distribution using the radiation dose values within the region different from the first and second shell structures (S₁, S₂), the metric of the predefined dose distribution used to determine which radiation dose values to be penalized.

12. A non-transitory computer-readable medium including computer code instructions stored thereon, the computer code instructions when executed cause one or more processors (**152**) to:
determine a first shell structure (S₁) defined around but not including a planning target volume, PTV, and having a first thickness;
determine a second shell structure (S₂) defined around but not including the first shell structure (S₁) and having a second thickness;
generate a first objective term of an objective function for optimizing a radiotherapy treatment plan, the first objective term penalizing radiation dose values in the first shell structure (S₁) exceeding a first radiation dose level specific to the first shell structure (S₁), the first objective term incorporating a first normal tissue objective, NTO, term, the first NTO term enforcing a first NTO within the first shell structure (S₁);
generate a second objective term of the objective function, the second objective term penalizing radiation dose values in the second shell structure (S₂) exceeding a second radiation dose level specific to the first shell structure (S₁), the second radiation dose level smaller than the first radiation dose level, the second objective term incorporating a second NTO term, the second NTO term enforcing the second NTO within the second shell structure (S₂);
generate a third objective term of the objective function, the third objective term penalizing radiation dose values in a region, different from the first and second shell structures (S₁, S₂), deviating from a predefined dose distribution; and
optimize the objective function to determine the radiotherapy treatment plan,
wherein the third objective term penalizes the radiation dose values in the region different from the first and second shell structures (S₁, S₂) exceeding a parameter of the predefined dose distribution and optionally
wherein the parameter of the predefined distribution includes:
a mean of the predefined distribution; or
a specific parameter of the predefined distribution.

## Patentansprüche

1. Verfahren zur Strahlenbehandlungsplanung, umfassend:
Bestimmen, durch einen oder mehrere Prozessoren (**152**), einer ersten Schalenstruktur (S₁), die um ein Planungszielvolumen, PTV, herum definiert ist, dieses aber nicht beinhaltet, und eine erste Dicke aufweist;
Bestimmen, durch den einen oder die mehreren Prozessoren (**152**), einer zweiten Schalenstruktur (S₂), die um die erste Schalenstruktur (S₁) herum definiert ist, diese aber nicht beinhaltet, und eine zweite Dicke aufweist;
Erzeugen, durch den einen oder die mehreren Prozessoren (**152**), eines ersten Zielfunktionsterms einer Zielfunktion zum Optimieren eines Strahlentherapiebehandlungsplans, wobei der erste Zielfunktionsterm Strahlendosiswerte in der ersten Schalenstruktur (S₁) bestraft, die ein erstes Dosisniveau überschreiten, das für die erste Schalenstruktur (S₁) spezifisch ist, wobei der erste Zielfunktionsterm einen ersten Normalgewebeziel-, NTO-, Term einbezieht, wobei der erste NTO-Term ein erstes NTO innerhalb der ersten Schalenstruktur (S₁) durchsetzt;
Erzeugen, durch den einen oder die mehreren Prozessoren (**152**), eines zweiten Zielfunktionsterms der Zielfunktion, wobei der zweite Zielfunktionsterm Strahlendosiswerte in der zweiten Schalenstruktur (S₂) bestraft, die ein zweites Dosisniveau überschreiten, das für die zweite Schalenstruktur (S₂) spezifisch ist, wobei das zweite Dosisniveau kleiner als das erste Dosisniveau ist, wobei der zweite Zielfunktionsterm einen zweiten NTO-Term einbezieht, wobei der zweite NTO-Term das zweite NTO innerhalb der zweiten Schalenstruktur (S₂) durchsetzt;
Erzeugen, durch den einen oder die mehreren Prozessoren (**152**), eines dritten Zielfunktionsterms der Zielfunktion, wobei der dritte Zielfunktionsterm Strahlendosiswerte in einer Region, die sich von der ersten und zweiten Schalenstruktur (S₁, S₂) unterscheidet, bestraft, die von einer vordefinierten Dosisverteilung abweichen; und
Optimieren, durch den einen oder die mehreren Prozessoren (**152**), der Zielfunktion zum Bestimmen des Strahlentherapiebehandlungsplans,
wobei die Region, die sich von der ersten und zweiten Schalenstruktur (S₁, S₂) unterscheidet, eine Normalgeweberegion außerhalb einer dritten Schalenstruktur (S₃) beinhaltet, die um das PTV herum definiert ist, und
wobei der dritte Zielfunktionsterm die Strahlendosiswerte in der Region, die sich von der ersten und zweiten Schalenstruktur (S₁, S₂) unterscheidet, bestraft, die einen Parameter der vordefinierten Dosisverteilung überschreiten, und wobei optional:
der Parameter der vordefinierten Verteilung beinhaltet:
einen Mittelwert der vordefinierten Verteilung; oder
einen spezifischen Parameter der vordefinierten Verteilung.

2. Verfahren nach Anspruch 1, wobei die zweite Dicke größer als die erste Dicke ist; oder
die erste Dicke gleich etwa 3 Millimeter ist; oder
die zweite Dicke gleich etwa 1,7 Zentimeter ist.

3. Verfahren nach einem der Ansprüche 1 oder 2, wobei das PTV ein erstes PTV ist und das Verfahren ferner umfasst:
Bestimmen, durch den einen oder die mehreren Prozessoren **(152)**, einer dritten Schalenstruktur, die um ein zweites PTV herum definiert ist, dieses aber nicht beinhaltet, und eine dritte Dicke aufweist;
Bestimmen, durch den einen oder die mehreren Prozessoren **(152)**, einer vierten Schalenstruktur, die um die dritte Schalenstruktur herum definiert ist, diese aber nicht beinhaltet, und eine vierte Dicke aufweist;
Erzeugen, durch den einen oder die mehreren Prozessoren **(152)**, eines vierten Zielfunktionsterms der Zielfunktion, wobei der vierte Zielfunktionsterm Strahlendosiswerte in der dritten Schalenstruktur bestraft, die ein drittes Strahlendosisniveau überschreiten, das für die dritte Schalenstruktur spezifisch ist; und
Erzeugen, durch den einen oder die mehreren Prozessoren **(152)**, eines fünften Zielfunktionsterms der Zielfunktion, wobei der fünfte Zielfunktionsterm Strahlendosiswerte in der vierten Schalenstruktur bestraft, die ein viertes Strahlendosisniveau überschreiten, das für die vierte Schalenstruktur spezifisch ist, wobei das vierte Strahlendosisniveau kleiner als das dritte Strahlendosisniveau ist.

4. Verfahren nach Anspruch 3, wobei die Region eine weitere Region außerhalb einer fünften Schalenstruktur, die um das erste PTV herum definiert ist, und außerhalb einer sechsten Schalenstruktur beinhaltet, die um das zweite PTV herum definiert ist,
die vordefinierte Dosisverteilung eine erste Dosisverteilung ist, die der fünften Schalenstruktur zugeordnet ist, und eine zweite Dosisverteilung der sechsten Schalenstruktur zugeordnet ist, und
der dritte Zielfunktionsterm Strahlendosiswerte in der weiteren Region bestraft, die von der ersten Dosisverteilung oder der zweiten Dosisverteilung abweichen.

5. Verfahren nach Anspruch 3 oder Anspruch 4, wobei sich zumindest die zweite Schalenstruktur (S₂) und die vierte Schalenstruktur überlappen und das Verfahren ferner umfasst:
Bestimmen, durch den einen oder die mehreren Prozessoren **(152)**, eines Überlappungsbereichs der zweiten und vierten Schalenstruktur;
Bestrafen, durch den einen oder die mehreren Prozessoren (**152**), von Strahlendosiswerten in dem Überlappungsbereich, die ein Maximum des zweiten Strahlendosisniveaus und des vierten Strahlendosisniveaus überschreiten.

6. Verfahren nach einem der Ansprüche 1 bis 5, wobei die Zielfunktion iterativ optimiert wird und wobei Erzeugen des dritten Zielfunktionsterms der Zielfunktion bei jeder Iteration beinhaltet:
Berechnen von Strahlendosiswerten innerhalb der Region, die sich von der ersten und zweiten Schalenstruktur (S₁, S₂) unterscheidet; und
Berechnen einer Metrik der vordefinierten Dosisverteilung unter Verwendung der Strahlendosiswerte innerhalb der Region, die sich von der ersten und zweiten Schalenstruktur (S₁, S₂) unterscheidet, wobei die Metrik der vordefinierten Dosisverteilung verwendet wird, um zu bestimmen, welche Strahlendosiswerte zu bestrafen sind.

7. Strahlenbehandlungsplanungssystem (**150**), umfassend:
einen oder mehrere Prozessoren (**152**); und
einen Speicher (**154**) zum Speichern von Computercodeanweisungen, wobei die Computercodeanweisungen bei Ausführung den einen oder die mehreren Prozessoren (**152**) veranlassen zum:
Bestimmen einer ersten Schalenstruktur (S₁), die um ein Planungszielvolumen, PTV, herum definiert ist, dieses aber nicht beinhaltet, und eine erste Dicke aufweist;
Bestimmen einer zweiten Schalenstruktur (S₂), die um die erste Schalenstruktur (S₁) herum definiert ist, diese aber nicht beinhaltet, und eine zweite Dicke aufweist;
Erzeugen eines ersten Zielfunktionsterms einer Zielfunktion zum Optimieren eines Strahlentherapiebehandlungsplans, wobei der erste Zielfunktionsterm Strahlendosiswerte in der ersten Schalenstruktur (S₁) bestraft, die ein erstes Strahlendosisniveau überschreiten, das für die erste Schalenstruktur (S₁) spezifisch ist, wobei der erste Zielfunktionsterm einen ersten Normalgewebeziel-, NTO-, Term einbezieht, wobei der erste NTO-Term ein erstes NTO innerhalb der ersten Schalenstruktur (S₁) durchsetzt;
Erzeugen eines zweiten Zielfunktionsterms der Zielfunktion, wobei der zweite Zielfunktionsterm Strahlendosiswerte in der zweiten Schalenstruktur (S₂) bestraft, die ein zweites Strahlendosisniveau überschreiten, das für die zweite Schalenstruktur (S₂) spezifisch ist, wobei das zweite Strahlendosisniveau kleiner als das erste Strahlendosisniveau ist, wobei der zweite Zielfunktionsterm einen zweiten NTO-Term einbezieht, wobei der zweite NTO-Term das zweite NTO innerhalb der zweiten Schalenstruktur (S₂) durchsetzt;
Erzeugen eines dritten Zielfunktionsterms der Zielfunktion, wobei der dritte Zielfunktionsterm Strahlendosiswerte in einer Region, die sich von der ersten und zweiten Schalenstruktur (S₁, S₂) unterscheidet, bestraft, die von einer vordefinierten Dosisverteilung abweichen; und
Optimieren der Zielfunktion zum Bestimmen des Strahlentherapiebehandlungsplans,
wobei der dritte Zielfunktionsterm die Strahlendosiswerte in der Region, die sich von der ersten und der zweiten Schalenstruktur (S₁, S₂) unterscheidet, die einen Parameter der vordefinierten Dosisverteilung überschreiten, bestraft und optional
wobei der Parameter der vordefinierten Verteilung beinhaltet:
einen Mittelwert der vordefinierten Verteilung; oder
einen spezifischen Parameter der vordefinierten Verteilung.

8. Strahlenbehandlungsplanungssystem (**150**) nach Anspruch 7, wobei die zweite Dicke größer als die erste Dicke ist und optional
wobei mindestens einer der folgenden Fälle zutrifft:
die erste Dicke gleich etwa 3 Millimeter ist; oder
die zweite Dicke gleich etwa 1,7 Zentimeter ist.

9. Strahlenbehandlungsplanungssystem (**150**) nach Anspruch 7 oder Anspruch 8, wobei das PTV ein erstes PTV ist und wobei der eine oder die mehreren Prozessoren (**152**) ferner konfiguriert sind zum:
Bestimmen einer dritten Schalenstruktur, die um ein zweites PTV herum definiert ist, dieses aber nicht beinhaltet, und eine dritte Dicke aufweist;
Bestimmen einer vierten Schalenstruktur, die um die dritte Schalenstruktur herum definiert ist, diese aber nicht beinhaltet, und eine vierte Dicke aufweist;
Erzeugen eines vierten Zielfunktionsterms der Zielfunktion zum Bestrafen von Strahlendosiswerten in der dritten Schalenstruktur, die ein drittes Strahlendosisniveau überschreiten, das für die dritte Schalenstruktur spezifisch ist; und
Erzeugen eines fünften Zielfunktionsterms der Zielfunktion, wobei der fünfte Zielfunktionsterm Strahlendosiswerte in der vierten Schalenstruktur bestraft, die ein viertes Strahlendosisniveau überschreiten, das für die vierte Schalenstruktur spezifisch ist, wobei das vierte Strahlendosisniveau kleiner als das dritte Strahlendosisniveau ist.

10. Strahlenbehandlungsplanungssystem (**150**) nach Anspruch 9, wobei die Region eine Region außerhalb einer fünften Schalenstruktur, die um das erste PTV herum definiert ist, und außerhalb einer sechsten Schalenstruktur, die um das zweite PTV herum definiert ist, beinhaltet,
die vordefinierte Dosisverteilung eine erste Dosisverteilung ist, die der fünften Schalenstruktur zugeordnet ist, und eine zweite Dosisverteilung der sechsten Schalenstruktur zugeordnet ist, und optional wobei das PTV ein erstes PTV ist und wobei der dritte Zielfunktionsterm Strahlendosiswerte in der Region, die von der ersten Dosisverteilung oder der zweiten Dosisverteilung abweichen, bestraft.

11. Strahlenbehandlungsplanungssystem (**150**) nach einem der Ansprüche 7 bis 10, wobei die Zielfunktion iterativ optimiert wird und wobei beim Erzeugen des dritten Zielfunktionsterms der Zielfunktion der eine oder die mehreren Prozessoren (**152**) konfiguriert sind, bei jeder Iteration:
Strahlendosiswerte innerhalb der Region, die sich von der ersten und der zweiten Schalenstruktur (S₁, S₂) unterscheidet, zu berechnen; und
eine Metrik der vordefinierten Dosisverteilung unter Verwendung der Strahlendosiswerte innerhalb der Region, die sich von der ersten und der zweiten Schalenstruktur (S₁, S₂) unterscheidet, zu berechnen, wobei die Metrik der vordefinierten Dosisverteilung verwendet wird, um zu bestimmen, welche Strahlendosiswerte zu bestrafen sind.

12. Nichtflüchtiges computerlesbares Medium, das darauf gespeicherte Computercodeanweisungen beinhaltet, wobei die Computercodeanweisungen bei Ausführung einen oder mehrere Prozessoren (**152**) veranlassen zum:
Bestimmen einer ersten Schalenstruktur (S₁), die um ein Planungszielvolumen, PTV, herum definiert ist, dieses aber nicht beinhaltet, und eine erste Dicke aufweist;
Bestimmen einer zweiten Schalenstruktur (S₂), die um die erste Schalenstruktur (S₁) herum definiert ist, diese aber nicht beinhaltet, und eine zweite Dicke aufweist;
Erzeugen eines ersten Zielfunktionsterms einer Zielfunktion zum Optimieren eines Strahlentherapiebehandlungsplans, wobei der erste Zielfunktionsterm Strahlendosiswerte in der ersten Schalenstruktur (S₁) bestraft, die ein erstes Strahlendosisniveau überschreiten, das für die erste Schalenstruktur (S₁) spezifisch ist, wobei der erste Zielfunktionsterm einen ersten Normalgewebeziel-, NTO-, Term einbezieht, wobei der erste NTO-Term ein erstes NTO innerhalb der ersten Schalenstruktur (S₁) durchsetzt;
Erzeugen eines zweiten Zielfunktionsterms der Zielfunktion, wobei der zweite Zielfunktionsterm Strahlendosiswerte in der zweiten Schalenstruktur (S₂) bestraft, die ein zweites Strahlendosisniveau überschreiten, das für die erste Schalenstruktur (S₁) spezifisch ist, wobei das zweite Strahlendosisniveau kleiner als das erste Strahlendosisniveau ist, wobei der zweite Zielfunktionsterm einen zweiten NTO-Term einbezieht, wobei der zweite NTO-Term das zweite NTO innerhalb der zweiten Schalenstruktur (S₂) durchsetzt;
Erzeugen eines dritten Zielfunktionsterms der Zielfunktion, wobei der dritte Zielfunktionsterm Strahlendosiswerte in einer Region, die sich von der ersten und zweiten Schalenstruktur (S₁, S₂) unterscheidet, bestraft, die von einer vordefinierten Dosisverteilung abweichen; und
Optimieren der Zielfunktion zum Bestimmen des Strahlentherapiebehandlungsplans,
wobei der dritte Zielfunktionsterm die Strahlendosiswerte in der Region, die sich von der ersten und der zweiten Schalenstruktur (S₁, S₂) unterscheidet, die einen Parameter der vordefinierten Dosisverteilung überschreiten, bestraft und optional
wobei der Parameter der vordefinierten Verteilung beinhaltet:
einen Mittelwert der vordefinierten Verteilung; oder
einen spezifischen Parameter der vordefinierten Verteilung.

## Revendications

1. Procédé de planification de traitement par rayonnement comprenant :
la détermination, par un ou plusieurs processeurs (**152**), d'une première structure de coque (S₁) définie autour mais ne comportant pas un volume cible de planification, PTV, et ayant une première épaisseur ;
la détermination, par le ou les processeurs (**152**), d'une deuxième structure de coque (S₂) définie autour mais ne comportant pas la première structure de coque (S₁) et ayant une deuxième épaisseur ;
la génération, par le ou les processeurs (**152**), d'un premier terme objectif d'une fonction objectif pour optimiser un plan de traitement de radiothérapie, le premier terme objectif pénalisant des valeurs de dose de rayonnement dans la première structure de coque (S₁) dépassant un premier niveau de dose spécifique à la première structure de coque (S₁), le premier terme objectif incorporant un premier terme objectif de tissu normal, NTO, le premier terme NTO imposant un premier NTO à l'intérieur de la première structure de coque (S₁) ;
la génération, par le ou les processeurs (**152**), d'un deuxième terme objectif de la fonction objectif, le deuxième terme objectif pénalisant des valeurs de dose de rayonnement dans la deuxième structure de coque (S₂) dépassant un deuxième niveau de dose spécifique à la deuxième structure de coque (S₂), le deuxième niveau de dose étant inférieur au premier niveau de dose, le deuxième terme objectif incorporant un second terme NTO, le second terme NTO imposant le second NTO à l'intérieur de la deuxième structure de coque (S₂) ;
la génération, par le ou les processeurs (**152**), d'un troisième terme objectif de la fonction objectif, le troisième terme objectif pénalisant les valeurs de dose de rayonnement dans une région, différente des première et deuxième structures de coque (S₁, S₂), s'écartant d'une distribution de dose prédéfinie ; et
l'optimisation, par le ou les processeurs (**152**), de la fonction objectif pour déterminer le plan de traitement de radiothérapie, dans lequel la région différente des première et deuxième structures de coque (S₁, S₂) comporte une région de tissu normal à l'extérieur d'une troisième structure de coque (S₃) définie autour du PTV, et
dans lequel le troisième terme objectif pénalise les valeurs de dose de rayonnement dans la région différente des première et deuxième structures de coque (S₁, S₂) dépassant un paramètre de la distribution de dose prédéfinie et dans lequel, éventuellement :
le paramètre de la distribution prédéfinie comporte :
une moyenne de la distribution prédéfinie ; ou
un paramètre spécifique de la distribution prédéfinie.

2. Procédé selon la revendication 1, dans lequel la deuxième épaisseur est supérieure à la première épaisseur ; ou
la première épaisseur est égale à environ 3 millimètres ; ou
la deuxième épaisseur est égale à environ 1,7 centimètres.

3. Procédé selon l'une quelconque de la revendication 1 ou de la revendication 2, dans lequel le PTV est un premier PTV et le procédé comprenant en outre :
la détermination, par le ou les processeurs **(152)**, d'une troisième structure de coque définie autour mais ne comportant pas un second PTV et ayant une troisième épaisseur ;
la détermination, par le ou les processeurs **(152)**, d'une quatrième structure de coque définie autour mais ne comportant pas la troisième structure de coque et ayant une quatrième épaisseur ;
la génération, par le ou les processeurs **(152)**, d'un quatrième terme objectif de la fonction objectif, le quatrième terme objectif pénalisant les valeurs de dose de rayonnement dans la troisième structure de coque dépassant un troisième niveau de dose de rayonnement spécifique à la troisième structure de coque ; et
la génération, par le ou les processeurs **(152)**, d'un cinquième terme objectif de la fonction objectif, le cinquième terme objectif pénalisant les valeurs de dose de rayonnement dans la quatrième structure de coque dépassant un quatrième niveau de dose de rayonnement spécifique à la quatrième structure de coque, le quatrième niveau de dose de rayonnement étant inférieur au troisième niveau de dose de rayonnement.

4. Procédé selon la revendication 3, dans lequel la région comporte une région supplémentaire à l'extérieur d'une cinquième structure de coque définie autour du premier PTV et à l'extérieur d'une sixième structure de coque définie autour du second PTV,
la distribution de dose prédéfinie est une première distribution de dose associée à la cinquième structure de coque, et une seconde distribution de dose est associée à la sixième structure de coque, et
le troisième terme objectif pénalise les valeurs de dose de rayonnement dans la région éloignée qui s'écartent de la première distribution de dose ou de la seconde distribution de dose.

5. Procédé selon la revendication 3 ou la revendication 4, dans lequel au moins la deuxième structure de coque (S₂) et la quatrième structure de coque se chevauchent, et le procédé comprenant en outre :
la détermination, par le ou les processeurs **(152)**, d'une région de chevauchement des deuxième et quatrième structures de coque ;
la pénalisation, par le ou les processeurs (**152**), de valeurs de dose de rayonnement dans la région de chevauchement dépassant un maximum du deuxième niveau de dose de rayonnement et du quatrième niveau de dose de rayonnement.

6. Procédé selon l'une quelconque des revendications 1 à 5, dans lequel la fonction objectif est optimisée de manière itérative, et dans lequel la génération du troisième terme objectif de la fonction objectif comporte, à chaque itération :
le calcul de valeurs de dose de rayonnement à l'intérieur de la région différente des première et deuxième structures de coque (S₁, S₂) ; et
le calcul d'une métrique de la distribution de dose prédéfinie à l'aide des valeurs de dose de rayonnement dans la région différente des première et deuxième structures de coque (S₁, S₂), la métrique de la distribution de dose prédéfinie étant utilisée pour déterminer quelles valeurs de dose de rayonnement doivent être pénalisées.

7. Système de planification de traitement par rayonnement (**150**) comprenant :
un ou plusieurs processeurs (**152**) ; et
une mémoire (**154**) pour stocker des instructions de code informatique, les instructions de code informatique, lorsqu'elles sont exécutées, amènent le ou les processeurs (**152**) à :
déterminer une première structure de coque (S₁) définie autour mais ne comportant pas un volume cible de planification, PTV, et ayant une première épaisseur ;
déterminer une deuxième structure de coque (S₂) définie autour mais ne comportant pas la première structure de coque (S₁) et ayant une deuxième épaisseur ;
générer un premier terme objectif d'une fonction objectif pour optimiser un plan de traitement de radiothérapie, le premier terme objectif pénalisant les valeurs de dose de rayonnement dans la première structure de coque (S₁) dépassant un premier niveau de dose de rayonnement spécifique à la première structure de coque (S₁), le premier terme objectif incorporant un premier terme objectif de tissu normal, NTO, le premier terme NTO imposant un premier NTO à l'intérieur de la première structure de coque (S₁) ;
générer un deuxième terme objectif de la fonction objectif, le deuxième terme objectif pénalisant les valeurs de dose de rayonnement dans la deuxième structure de coque (S₂) dépassant un deuxième niveau de dose de rayonnement spécifique à la deuxième structure de coque (S₂), le deuxième niveau de dose de rayonnement étant inférieur au premier niveau de dose de rayonnement, le deuxième terme objectif incorporant un second terme NTO, le second terme NTO imposant le second NTO à l'intérieur de la deuxième structure de coque (S₂) ;
générer un troisième terme objectif de la fonction objectif, le troisième terme objectif pénalisant les valeurs de dose de rayonnement dans une région, différente des première et deuxième structures de coque (S₁, S₂), s'écartant d'une distribution de dose prédéfinie ; et
optimiser la fonction objectif pour déterminer le plan de traitement de radiothérapie,
dans lequel le troisième terme objectif pénalise les valeurs de dose de rayonnement dans la région différente des première et deuxième structures de coque (S₁, S₂) dépassant un paramètre de la distribution de dose prédéfinie et éventuellement
dans lequel le paramètre de la distribution prédéfinie comporte :
une moyenne de la distribution prédéfinie ; ou
un paramètre spécifique de la distribution prédéfinie.

8. Système de planification de traitement de radiothérapie (**150**) selon la revendication 7, dans lequel la deuxième épaisseur est supérieure à la première épaisseur et, éventuellement
dans lequel au moins une des conditions suivantes s'applique :
la première épaisseur est égale à environ 3 millimètres ; ou
la deuxième épaisseur est égale à environ 1,7 centimètres.

9. Système de planification de traitement de radiothérapie (**150**) selon la revendication 7 ou la revendication 8, dans lequel le PTV est un premier PTV et dans lequel le ou les processeurs (**152**) sont en outre configurés pour :
déterminer une troisième structure de coque définie autour mais ne comportant pas un second PTV et ayant une troisième épaisseur ;
déterminer une quatrième structure de coque définie autour mais ne comportant pas la troisième structure de coque et ayant une quatrième épaisseur ;
générer un quatrième terme objectif de la fonction objectif pour pénaliser les valeurs de dose de rayonnement dans la troisième structure de coque dépassant un troisième niveau de dose de rayonnement spécifique à la troisième structure de coque ; et
générer un cinquième terme objectif de la fonction objectif, le cinquième terme objectif pénalisant les valeurs de dose de rayonnement dans la quatrième structure de coque dépassant un quatrième niveau de dose de rayonnement spécifique à la quatrième structure de coque, le quatrième niveau de dose de rayonnement étant inférieur au troisième niveau de dose de rayonnement.

10. Système de planification de traitement de radiothérapie (**150**) selon la revendication 9, dans lequel la région comporte une région à l'extérieur d'une cinquième structure de coque définie autour du premier PTV et à l'extérieur d'une sixième structure de coque définie autour du second PTV,
la distribution de dose prédéfinie est une première distribution de dose associée à la cinquième structure de coque, et une seconde distribution de dose est associée à la sixième structure de coque, et éventuellement dans lequel le PTV est un premier PTV et dans lequel le troisième terme objectif pénalise les valeurs de dose de rayonnement dans la région s'écartant de la première distribution de dose ou de la seconde distribution de dose.

11. Système de planification de traitement de radiothérapie (**150**) selon l'une quelconque des revendications 7 à 10, dans lequel la fonction objectif est optimisée de manière itérative, et dans lequel, lors de la génération du troisième terme objectif de la fonction objectif, le ou les processeurs (**152**) sont configurés pour, à chaque itération :
calculer les valeurs de dose de rayonnement à l'intérieur de la région différente des première et deuxième structures de coque (S₁, S₂) ; et
calculer une métrique de la distribution de dose prédéfinie à l'aide des valeurs de dose de rayonnement dans la région différente des première et deuxième structures de coque (S₁, S₂), la métrique de la distribution de dose prédéfinie étant utilisée pour déterminer quelles valeurs de dose de rayonnement doivent être pénalisées.

12. Support non transitoire lisible par ordinateur comportant des instructions de code informatique stockées sur celui-ci, les instructions de code informatique, lorsqu'elles sont exécutées, amènent un ou plusieurs processeurs (**152**) à :
déterminer une première structure de coque (S₁) définie autour mais ne comportant pas un volume cible de planification, PTV, et ayant une première épaisseur ;
déterminer une deuxième structure de coque (S₂) définie autour mais ne comportant pas la première structure de coque (S₁) et ayant une deuxième épaisseur ;
générer un premier terme objectif d'une fonction objectif pour optimiser un plan de traitement de radiothérapie, le premier terme objectif pénalisant les valeurs de dose de rayonnement dans la première structure de coque (S₁) dépassant un premier niveau de dose de rayonnement spécifique à la première structure de coque (S₁), le premier terme objectif incorporant un premier terme objectif de tissu normal, NTO, le premier terme NTO imposant un premier NTO à l'intérieur de la première structure de coque (S₁) ;
générer un deuxième terme objectif de la fonction objectif, le deuxième terme objectif pénalisant les valeurs de dose de rayonnement dans la deuxième structure de coque (S₂) dépassant un deuxième niveau de dose de rayonnement spécifique à la première structure de coque (S₁), le deuxième niveau de dose de rayonnement étant inférieur au premier niveau de dose de rayonnement, le deuxième terme objectif incorporant un second terme NTO, le second terme NTO imposant le second NTO à l'intérieur de la deuxième structure de coque (S₂) ;
générer un troisième terme objectif de la fonction objectif, le troisième terme objectif pénalisant les valeurs de dose de rayonnement dans une région, différente des première et deuxième structures de coque (S₁, S₂), s'écartant d'une distribution de dose prédéfinie ; et
optimiser la fonction objectif pour déterminer le plan de traitement de radiothérapie,
dans lequel le troisième terme objectif pénalise les valeurs de dose de rayonnement dans la région différente des première et deuxième structures de coque (S₁, S₂) dépassant un paramètre de la distribution de dose prédéfinie et éventuellement
dans lequel le paramètre de la distribution prédéfinie comporte :
une moyenne de la distribution prédéfinie ; ou
un paramètre spécifique de la distribution prédéfinie.
